# EUROPEAN PATENT SPECIFICATION

(11) **EP 3 365 068 B1**
(45) Date of publication and mention of the grant of the patent: **01.04.2020**
(21) Application number: 16782299.8
(22) Date of filing: 04.10.2016
(51) Int. Cl.: A61Q 19/02, A61K 8/9789

(54) **COSMETIC COMPOSITIONS FOR SKIN WHITENING**
KOSMETISCHE ZUSAMMENSETZUNGEN ZUR HAUTBLEICHUNG
COMPOSITIONS COSMÉTIQUES POUR BLANCHIMENT DE PEAU

(30) Priority: 22.10.2015 FI 20155752
(43) Date of publication of application: 29.08.2018
(73) Proprietor: Lumene Oy, 02780 Espoo (FI)
(72) Inventor: BACKMAN, Josefin, 10300 Karis (FI); ISOHANNI, Tiina, 02400 Kirkkonummi (FI)
(74) Representative: Laine IP Oy
(86) International application number: PCT/FI2016/050688
(87) International publication number: WO 2017/068234

(56) References cited:
- EP-A1- 1 073 446
- WO-A1-2013/124540
- WO-A2-2009/125071
- DE-A1-102008 019 340
- US-A1- 2011 123 463
- US-A1- 2012 156 296
- US-A1- 2013 172 291
- Kiantama: "Berry Powders and Crushed Berries", , 2011, XP002764615, Retrieved from the Internet: URL:http://www.kiantama.fi/en/products/ber ry-powders-and-crushed-berries/ [retrieved on 2016-11-23]

## Description

### Field of the Invention

The present invention relates to a cosmetic composition containing spray-dried powder derived from cloudberry (*Rubus chamaemorus*) fruit. Said powder finds use especially in skin-whitening compositions, including emulsion creams, foundation creams and skin serums.

### Background of the Invention

Various cosmetic treatments are provided in the art in order to minimize pigment-related skin problems such as skin discoloration or formation of age spots, freckles and melasma.

An important factor in skin pigmentation is the production of melanin in the skin melanocytes. Melanin production involves tyrosine, which is converted to DOPA, then to dopa-quinone by the catalytic action of tyrosinase, and further intermediate products. Melanin exists in the skin and protects the body from, for instance, UV rays, and has an important function in the regulation of internal secretion of hormones.

Multiple of substances such as vitamin C, kojic acid, arbutin and hydroquinone are currently used in skin whitening products. However, at least kojic acid and hydroquinone may cause skin irritation or sensitivity, and thus they may not be optimal substances for skin whitening compositions.

Use of plant extracts in skin whitening products has also been suggested. For instance, US patent application No. 2012/0213719, discloses a cosmetic composition for skin whitening, which comprises extracts derived from Magnolia, Citrus, wild soybean, Ginko or Honeysuckle plants. The composition may further comprise skin whitening compounds, such as arbutin, ascorbic acid or kojic acid. In US patent application No. 2014/0271509 a skin whitening composition is disclosed, which comprises tyrosinase-inhibiting compounds and plant extracts which inhibit α-melanocyte stimulating hormone. The composition may also contain hydroquinone derivatives.

A variety of plants, even some berries contain ellagic acid in free form or in the form of ellagitannin glycosides. These compounds have been reported to have inhibitory effect on melanin formation by suppressing tyrosinase activity (1). It was shown by Shimogaki *et al.* (2), that ellagic acid inhibits tyrosinase activity by reacting with the copper present at the active site of the enzyme.

Cosmetic compositions derived from cloudberry fruit are currently in use. However, there are no indications in prior art that a cloudberry-derived preparation would have skin whitening characteristics.

### Summary of the Invention

The present invention discloses a cosmetic composition, which comprises cosmetically acceptable substances and, as an active ingredient, spray-dried powder obtained by spray drying a mixture comprising cloudberry juice and maltodextrin, and additionally cloudberry seed oil and/or cloudberry seed extract.

### Detailed Description of the Invention

The cosmetic composition disclosed in the present specification comprises spray-dried powder, which has been prepared from cloudberry juice and maltodextrin. The composition of the invention may also comprise other cosmetically acceptable whitening agents, and further comprises cloudberry seed oil and/or cloudberry seed extract.

The mode of action of the powder used in the present invention is very versatile, since it inhibits both
- the enzymatic activity of tyrosinase,
- melanin synthesis, and
- dendricity of normal human epidermal melanocytes.

Consequently, the powder has skin whitening properties and finds use in treating pigment-related skin problems. It can thus be anticipated that the composition of the invention fades away or minimizes appearance of dark spots, age spots, liver spots, and freckles, and evens out skin tone and inhibits future pigment formation.

The prior known whitening agents are not as many-sided. For instance, the action of hydroquinone, kojic acid and arbutin is mainly related to the tyrosinase enzyme. Hydroquinone is a strong inhibitor of melanin production by occupying the active site of the tyrosinase enzyme, kojic acid is able to prevent tyrosinase activity by binding to copper, and arbutin inhibits the tyrosinase activity, but does not affect the tyrosinase gene expression. Vitamin C, on the other hand, interacts with copper ions to reduce the formation of dopa-quinone.

The spray-dried powder may be prepared simply by pressing the juice from cloudberry fruit, filtering and mixing the juice with maltodextrin, and then spray-drying the mixture. A preferable ratio is 30% of cloudberry juice and 70% of maltodextrin. All percentages given in this specification are per weight, if not otherwise indicated. For the purposes of the invention, such a powder is named as "spray-dried cloudberry powder 30%". The powder so obtained may be used in the cosmetic compositions of the present invention. The concentration of the powder in a typical composition is 0.001 - 30% of the total weight of the composition.

The composition of the invention is supposed to be used topically. The formulations of the composition include but are not restricted to skin lotion, skin softener, skin toner, lotion, ointment, milk lotion, moisture lotion, nutrition lotion, massage cream, nutrition cream, emulsion cream, moisture cream, hand cream, skin serum, essence, nutrition essence, cleansing foam, cleansing lotion, cleansing cream, body lotion, body cleanser, makeup base, foundation cream, pressed powder, and loose powder.

The chemical analyses of the spray-dried cloudberry powder showed that it contains same amounts of total phenolics and ellagitannins as cloudberry fruit. The amount of phenolic acids and Vitamin C in the spray-dried powder was about 1/10 compared to cloudberry fruit. The antioxidant activity for the spray-dried powder was slightly higher than that for cloudberry fruit. On the other hand, the powder contained only traces of alpha-tocopherol, phytosterols and oil compared to cloudberry fruit, and no carotenoids were detected.

As indicated, the antioxidant activity of the powder is high, which is mainly due to the high amounts of total phenolics, ellagitannins and Vitamin C.

It should be noted that the term skin whitening may be used commensurate with the terms skin brightening, skin lightening, but also skin bleaching. Skin radiance is also a term used in the cosmetic field when referring to improvement of skin appearance.

Tyrosinase is required for melanocytes to produce melanin from the amino acid tyrosine. Since tyrosinase is the first limiting enzyme of the melanin synthesis pathway, specific inhibitors of this enzyme may thus induce inhibition of melanin synthesis and subsequently whitening effects. Therefore, in the evaluation of whitening effects of the spray-dried powder of the present invention the first step was to measure the activity of tyrosinase enzyme extracted from normal human epidermal melanocytes (NHEM), using an acellular assay. As a reference kojic acid was used.

Two further assays were carried out to evaluate the whitening effect of the powder, i.e. melanin synthesis in NHEM stimulated by L-tyrosinase and melanocyte dendricity in a co-culture of NHEM and normal human epidermal keratinocytes (NHEK) in presence of IBMX (a melanocyte dendricity stimulating agent) using *in situ* immunofluorescent labeling and image analysis. As reference substances in these tests lipoic acid and vincristine, respectively, were used.

The spray-dried cloudberry powder according to the present invention inhibited the enzymatic activity of tyrosinase, melanin synthesis and dendricity of normal human epidermal melanocytes. These results indicate that the powder has whitening properties.

### Experimental

### Example 1. Preparation of the spray-dried cloudberry powder

Cloudberries (*Rubus chamaemorus*) were collected from a forest in northern Finland. The berries were warmed and treated with pectinase, and the juice was pressed using a conventional presser. Subsequently, the juice was filtered, pasteurized and concentrated. The concentrate was diluted with water and mixed with maltodextrin in a ratio of 30% of cloudberry juice and 70% of maltodextrin (per weight) and the mixture was spray-dried in a conventional spray-drier, by leading the concentrated mixture into the drying chamber as spray, whereby water was evaporated and the dried particles separated. The powder was cooled down, sieved out and packed. The powder so obtained was named as "spray-dried cloudberry powder 30%".

The powder was chemically analyzed as described in Example 2, and its whitening effect was evaluated according to Example 3.

### Example 2. Chemical analysis of the spray-dried cloudberry powder

Chemical analyses were performed on the spray-dried cloudberry powder as prepared according to Example 1 (containing 30 % of cloudberry juice and 70% of maltodextrin).

The following analyses were performed:
Total phenolics by Folin-Ciocalteu method, concentration of ellagitannins (by HPLC-QTOF-MS), HPLC-profiles of phenolic compounds and quantification of phenolic acids, concentration of Vitamin C as dehydroascorbic acid by HPLC with fluorescence detection, concentration of Vitamin E and carotenoids by HPLC after saponification, concentration of phytosterols by GC-MS, and concentration of fatty acids by GC-MS.

The analyses showed that the spray-dried cloudberry powder contained same amounts of total phenolics as cloudberry fruit (3). Also, the amount of ellagitannins in the spray-dried cloudberry powder was the same as in cloudberry fruit (4). The amount of phenolic acids in the spray-dried powder was about 1/10 compared to cloudberry fruit (5). The antioxidant activity for the spray-dried powder was slightly higher than that for cloudberry fruit (6). The amount of Vitamin C in the spray-dried powder was 975 µg/g (measured as dehydroascorbic acid). This is about 1/10 of the amount found in cloudberry fruit. The spray-dried powder contained only traces of alpha-tocopherol compared to cloudberry fruit, and no carotenoids were detected. The spray-dried powder contained only traces of phytosterols and oils compared to cloudberry fruit (7).

### Example 3. Evaluation of the whitening effect of spray-dried cloudberry powder

The whitening effect of spray-dried cloudberry powder was evaluated by measuring the enzymatic activity of tyrosinase, melanin synthesis and the melanocyte dendricity in a model of normal human epidermal melanocytes (NHEM).

More specifically, the effect of the compound was evaluated on the following parameters:
1. Activity of tyrosinase enzyme extracted from NHEM (acellular assay).
2. Melanin synthesis in NHEM stimulated by L-tyrosinase.
3. Melanocyte dendricity in a co-culture of NHEM and normal human epidermal keratinocytes (NHEK) in presence of IBMX (a melanocyte dendricity stimulating agent) using *in situ* immunofluorescent labeling and image analysis.

The first part of the study consisted of testing the solubility of spray-dried cloudberry powder 30% and evaluating its effects on the morphology of human melanocytes. The optimal concentration of the compound that was tolerated by the cells was determined.

### Cytotoxicity preliminary assay

The test was performed on NHEM in culture medium and the incubation time was 72 hours. The evaluation parameters were MTT reduction assay and morphological observations with a microscope. The results of the MTT reduction assay and the observation of the cell layers determined the concentrations to be tested. (MTT = 3-(4,5-dimethyl-2-thiazolyl)-2,5-diphenyl-2H-tetrazolium bromide.)

For the tyrosinase activity assay the following concentrations were tested: 0.33, 1, 3, 9, 27 and 81 mg/ml (the spray-dried cloudberry powder was dissolved in assay buffer). For the melanin synthesis and melanocyte dendricity assay the following concentrations were tested: 0.33, 1 and 3 mg/ml (the spray-dried cloudberry powder was dissolved in culture medium).

### 1. Tyrosinase enzymatic activity

The enzyme tyrosinase was extracted from normal human epidermal melanocytes. The substrate used was 3,4-dihydroxy-phenylalanine (L-DOPA).

The spray-dried powder or the reference substance (kojic acid tested at 5 concentrations from 0.0089 mg/ml to 0.142 mg/ml) was pre-incubated with the enzyme for 10 minutes on ice. At the end of the incubation, the substrate, L-DOPA was added and the plates were incubated at 37°C for 1 hour. The enzymatic activity was evaluated by measuring the optical density (OD) at 540 nm. Measurements of optical density were also performed without substrate addition in order to detect compound or reference interference for each tested concentration. All experimental conditions were performed in n=3. The results were expressed as percentage of activity compared to control and % of inhibition.

### Effect on enzymatic activity of tyrosinase:

The reference, kojic acid, tested from 0.0089 mg/ml to 0.142 mg/ml, induced a strong concentration-dependent inhibition of the enzymatic activity of tyrosinase extracted from human melanocytes (estimated IC₅₀ of 0.4 mM). This result was expected and validated the assay.

The spray-dried cloudberry powder 30%, tested from 0.33 mg/ml to 81 mg/ml, induced a strong concentration-dependent inhibition of tyrosinase enzymatic activity, with a maximal effect observed as soon as 9 mg/ml. The estimated IC₅₀ was 3.7 mg/ml.

### 2. Melanin synthesis under stimulated condition

Melanocytes were seeded in 24-well plates and cultured in their culture medium for 24 hours. The medium was then replaced by culture medium containing or not (stimulated control) the inducer (L-Tyrosine) and the test compound of the reference (lipoic acid at 5 µg/ml). The cells were then incubated for 10 days with treatment renewal every 3 or 4 days.

In parallel, a non-stimulated control was performed. Each experimental condition was performed in n=3.

At the end of incubation, melanin was extracted by cell lysis using a 0.5 N NaOH solution. The optical density (OD) of each experimental point was measured at 405 nm and melanin quantity was calculated using melanin standards. Results were expressed in µg/ml of melanin and in percentage of inhibition compared to control.

### Effect on melanin synthesis:

The treatment with L-tyrosine, tested at 1 mM, resulted in a significant stimulating effect on melanin synthesis by normal human epidermal melanocytes (NHEM). The reference, lipoic acid, tested at 5 µg/ml, showed an inhibitory effect on L-tyrosine-induced melanin synthesis (108% of inhibition). These results were expected and validated the assay.

The spray-dried cloudberry powder 30% tested at 0.33, 1 and 3 mg/ml induced a concentration-dependent inhibition of L-tyrosine-induced melanin synthesis (46% of inhibition at the highest test concentration).

### 3. Melanocyte dendricity under stimulated condition

Normal human epidermal melanocytes and normal human epidermal keratinocytes (NHEM+NHEK) were seeded in 96-well plates and cultured in their culture medium for 24 hours. The medium was then replaced by culture medium containing or not (stimulated control) the inducer (IBMX) and the test compound of the references (vincristine at 0.025 µg/ml). The cells were then incubated for 72 hours.

In parallel, a non-stimulated control was performed. Each experimental condition was performed in n=3.

After incubation, culture media were discarded and the cells were rinsed, fixed and permeabilized. The cells were then labeled using a primary antibody anti-Mel5. The primary antibody was then revealed using a fluorescent secondary antibody (GAM-Alexa 488) and the cell nuclei were stained using Hoechst 33258 solution in parallel.

Images were acquired of the wells (10 photos per well). The labeling was quantified by the measurement of the dendrite fluorescence area normalized to the total number of melanocytes.

### Effect on melanocyte dendricity:

The treatment of the NHEM/NHEK co-culture with IBMX, tested at 200 µM, clearly stimulated the formation of dendrites by the normal human epidermal melanocytes (NHEM). The reference vincristine, tested at 0.025 µg/ml, showed an inhibitory effect on the formation of dendrites by the melanocytes (175% of inhibition). These results were expected and validated the assay.

The spray-dried cloudberry powder 30% tested at 3 mg/ml induced a strong inhibition of NHEM dendricity (135% of inhibition).

### Conclusions:

Under the experimental conditions of this study, the spray-dried cloudberry powder 30% according to the present invention inhibited the enzymatic activity of tyrosinase, melanin synthesis and dendricity of normal human epidermal melanocytes. These results indicate that the powder has whitening properties.

### Example 4. Preparation of an emulsion cream

An example of ingredients for a ready-for-use emulsion cream composition (Day Cream) for facial and throat skin care containing spray-dried cloudberry powder is shown in the following Table 1. The described emulsion cream composition moisturizes, brightens and softens the skin. In addition, the emulsion cream according to the invention possesses antioxidant properties.

**Table 1. Day Cream.**

| INGREDIENTS | Ratio % |
|---|---|
| Aqua (Water) | Add 100 |
| Steareth-2 | 4.0-5.0 |
| Dimethicone | 3.0-5.0 |
| Glycerin | 2.0-5.0 |
| Steareth-21 | 2.500 |
| Octyldodecanol | 1.0-3.0 |
| Stearic Acid | 1.0-3.0 |
| Spray-dried Cloudberry Powder | 0.001-30 |
| Cetearyl Alcohol | 1.500 |
| Heptyl Undecylenate | 1.0-2.0 |
| Caprylic/Capric Triglyceride | 1.000 |
| PEG-30 Dipolyhydroxystearate | 0.5-1.0 |
| Aluminum Starch Octenyl Succinate | 0.5-1.0 |
| Phenoxyethanol | 0.5-1.0 |
| Cetearyl Dimethicone Crosspolymer | 0.5-1.0 |
| Tocopheryl Acetate | 0.2-0.8 |
| PPG-15 Stearyl Ether | 0.3-0.6 |
| Parfum (Fragrance) | 0.390 |
| Lecithin | 0.203 |
| Xanthan Gum | 0.15-0.2 |
| Acrylates/C10-30 Alkyl Acrylate Crosspolymer | 0.100 |
| Disodium EDTA | 0.100 |
| Magnesium Ascorbyl Phosphate | 0.100 |

### Example 5. Preparation of a foundation cream

An example of ingredients for a foundation cream composition containing spray-dried cloudberry powder for facial skin is presented in Table 2. The cream moisturizes the skin, and protects it from UV-radiation. The cream also gives the skin light coverage and colour.

**Table 2. Foundation Cream**

| INGREDIENTS | Ratio % |
|---|---|
| Aqua (Water) | 50.0-55.0 |
| Cyclopentasiloxane | 10.0-15.0 |
| (CI 77891) Titanium Dioxide | 5.0-10.0 |
| Cyclohexasiloxane | 3.0-5.0 |
| Isododecane | 2.0-10.0 |
| Ethylhexyl Methoxycinnamate (Octinoxate) | 1.0-6.0 |
| Polyglyceryl-4 isostearate | 1.0-2.0 |
| Magnesium Sulfate | 1.0-2.0 |
| Cetyl PEG/PPG-10/1 Dimethicone | 1.0-2.0 |
| Hexyl laurate | 1.0-2.0 |
| (CI 77491) Iron Oxides | 1.0-2.0 |
| MICA | 1.0-2.0 |
| Nylon-12 | 0.5-1.0 |
| Propylene Glycol | 0.5-1.0 |
| Titanium Dioxide | 0.5-1.0 |
| Phenoxyethanol | 0.5-1.0 |
| Disteardimonium Hectorite | 0.5-1.0 |
| Silica | 0.5-1.0 |
| (CI 77492) Iron Oxides | 0.1-1.0 |
| Butylene Glycol | 0.1-1.0 |
| Spray-dried Cloudberry Powder | 0.001-30 |
| (CI 77499) Iron Oxides | 0.1-1.0 |
| Perfluorooctyl Triethoxysilane | 0.1-1.0 |
| Propylene Carbonate | 0.05-0.3 |
| Dimethicone Crosspolymer | 0.05-0.3 |
| Methicone | 0.05-0.3 |
| Parfum (Fragrance) | 0.05-0.3 |
| Ethylhexylglycerin | 0.05-0.3 |
| Alumina | 0.05-0.3 |
| Triethoxycaprylylsilane | 0.05-0.3 |
| | 100.001 |

### Example 6. Preparation of a serum composition

An example of ingredients for a serum composition containing spray-dried cloudberry powder is presented in Table 3.

**Table 3. Serum composition.**

| INGREDIENTS | Ratio % |
|---|---|
| Aqua (Water) | Add 100 |
| Heptyl Undecylenate | 4.000 |
| Methylpropanediol | 3.000 |
| Glycerin | 3.000 |
| Spray-dried Cloudberry Powder | 0.001-30 |
| Phenoxyethanol | 1.000 |
| Hydroxyethyl Acrylate/Sodium Acryloldimethyl Taurate Copolymer | 0.300 |
| Xantan Gum | 0.1-0.4 |
| PVP | 0.200 |
| Ethylhexylglycerin | 0.200 |
| Propanediol | 0.163 |
| Ammonium Acryloyldimethyltaurate/ VP Copolymer | 0.150 |
| Caprylyl Glycol | 0.150 |
| Polyisobutene | 0.150 |
| Ethylhexyl Cocoate | 0.100 |
| Disodium EDTA | 0.100 |
| Lecithin | 0.056 |
| PEG-7 Trimethylolpropane Coconut Ether | 0,025 |
| Glucose | 0.008 |
| Peat Extract | 0.007 |
| Chondrus Crispus (Carrageenan) | 0.002 |
| Glycolipids | 0.000 |

### References

(1) Özer et al., Pharmaceutical Biology, 2007, 45 (6), 519-524.
(2) Shimogaki et al., International Journal of Cosmetic Science, 2000, 22, 291-303
(3) VTT. Earlier published results
(4) Arctic Flavours Association, www.arctic-flavours.fi or Koponen et al. J. Agric. Food Chem., 2007, 55 (4), pp 1612-1619
(5) Arctic Flavours Association, www.arctic-flavours.fi
(6) VTT. Earlier published results
(7) Fineli® - Finnish Food Composition Database

## Claims

1. A cosmetic composition for skin whitening, which comprises cosmetically acceptable substances, **characterized in that** it contains spray-dried powder obtained by spray drying a mixture comprising cloudberry juice and maltodextrin, and **in that** the composition further comprises cloudberry seed oil and/or cloudberry seed extract.

2. The cosmetic composition according to claim 1, **characterized in that** the mixture comprises 30% of cloudberry juice and 70% of maltodextrin.

3. The cosmetic composition according to claim 1 or 2, **characterized in that** the composition is formulated as a cream, serum, lotion, ointment or powder.

4. The cosmetic composition according to any one of claims 1-3, **characterized in that** the concentration of the powder in a typical composition is 0.001 - 30% of the total weight of the composition.

5. The cosmetic composition according to any one of claims 1-4, **characterized in that** the composition further comprises at least one substance selected from cosmetically acceptable whitening agents.

6. Use of spray-dried powder obtained by spray drying a mixture comprising cloudberry juice and maltodextrin for skin whitening.

7. A method for whitening skin, comprising applying a cosmetic composition comprising spray-dried powder obtained by spray drying a mixture comprising cloudberry juice and maltodextrin to a portion of skin.

## Patentansprüche

1. Kosmetische Zusammensetzung zur Hautbleichung, die kosmetisch akzeptable Substanzen umfasst, **dadurch gekennzeichnet, dass** sie sprühgetrocknetes Pulver enthält, das erhalten wird, indem ein Gemisch, das Moltebeerensaft und Maltodextrin umfasst, sprühgetrocknet wird, und dadurch, dass die Zusammensetzung weiter Moltebeerensamenöl und/oder Moltebeerensamenextrakt umfasst.

2. Kosmetische Zusammensetzung nach Anspruch 1, **dadurch gekennzeichnet, dass** das Gemisch 30% Moltebeerensaft und 70% Maltodextrin umfasst.

3. Kosmetische Zusammensetzung nach Anspruch 1 oder 2, **dadurch gekennzeichnet, dass** die Zusammensetzung als ein(e), Creme, Serum, Lotion, Salbe oder Puder formuliert ist.

4. Kosmetische Zusammensetzung nach einem der Ansprüche 1-3, **dadurch gekennzeichnet, dass** die Konzentration des Pulvers in einer typischen Zusammensetzung 0,001 - 30% des Gesamtgewichts der Zusammensetzung ist.

5. Kosmetische Zusammensetzung nach einem der Ansprüche 1-4, **dadurch gekennzeichnet, dass** die Zusammensetzung weiter mindestens eine Substanz umfasst, die aus kosmetisch annehmbaren Bleichmitteln ausgewählt ist.

6. Verwendung von sprühgetrocknetem Pulver, das durch Sprühtrocknen eines Gemisches erhalten wird, das Moltebeerensaft und Maltodextrin umfasst, zur Hautbleichung.

7. Verfahren zur Hautbleichung, umfassend Auftragen einer kosmetischen Zusammensetzung, die sprühgetrocknetes Pulver umfasst, das durch Sprühtrocknen eines Gemisches erhalten wird, das Moltebeerensaft und Maltodextrin umfasst, auf einen Hautbereich.

## Revendications

1. Composition cosmétique pour blanchiment de la peau, qui comprend des substances cosmétiquement acceptables, **caractérisée en ce qu'**elle contient de la poudre séchée par pulvérisation obtenue en séchant par pulvérisation un mélange comprenant du jus de ronce petit-mûrier et de la maltodextrine, et **en ce que** la composition comprend en outre de l'huile de graines de ronce petit-mûrier et/ou de l'extrait de graines de ronce petit-mûrier.

2. Composition cosmétique selon la revendication 1, **caractérisée en ce que** le mélange comprend 30 % de jus de ronce petit-mûrier et 70 % de maltodextrine.

3. Composition cosmétique selon la revendication 1 ou 2, **caractérisée en ce que** la composition est formulée sous forme de crème, de sérum, de lotion, de pommade ou de poudre.

4. Composition cosmétique selon l'une quelconque des revendications 1 à 3, **caractérisée en ce que** la concentration de la poudre dans une composition classique est de 0,001 à 30 % du poids total de la composition.

5. Composition cosmétique selon l'une quelconque des revendications 1 à 4, **caractérisée en ce que** la composition comprend en outre au moins une substance sélectionnée parmi des agents de blanchiment cosmétiquement acceptables.

6. Utilisation de poudre séchée par pulvérisation obtenue en séchant par pulvérisation un mélange comprenant du jus de ronce petit-mûrier et de la maltodextrine pour blanchiment de la peau.

7. Procédé pour blanchiment de la peau, comprenant l'application d'une composition cosmétique comprenant de la poudre séchée par pulvérisation obtenue en séchant par pulvérisation un mélange comprenant du jus de ronce petit-mûrier et de la maltodextrine à une partie de la peau.
